# EUROPEAN PATENT SPECIFICATION

(11) **EP 2 550 032 B1**
(45) Date of publication and mention of the grant of the patent: **20.11.2013**
(21) Application number: 11709730.3
(22) Date of filing: 23.03.2011
(51) Int. Cl.: A61L 31/02, A61L 31/14, C22C 23/00, C22C 23/06, A61F 2/82

(54) **IMPLANT MADE OF A BIODEGRADABLE MAGNESIUM ALLOY**
IMPLANTAT AUS EINER BIOLOGISCH ABBAUBAREN MAGNESIUMLEGIERUNG
IMPLANT CONSTITUÉ PAR UN ALLIAGE DE MAGNÉSIUM BIODÉGRADABLE

(30) Priority: 25.03.2010 US 317296 P
(43) Date of publication of application: 30.01.2013
(73) Proprietor: Biotronik AG, 8180 Bülach (CH)
(72) Inventor: GEROLD, Bodo, 91225 Zellingen (DE)
(74) Representative: Lindner-Vogt, Karin L.
(86) International application number: PCT/EP2011/054448
(87) International publication number: WO 2011/117298

(56) References cited:
- EP-A1- 2 000 551
- CN-A- 101 078 080
- DE-A1-102004 043 231

## Description

The present invention relates to implants made of a biodegradable magnesium alloy.

Medical implants for greatly varying uses are known in the art. A shared goal in the implementation of modern medical implants is high biocompatibility, i.e., a high degree of tissue compatibility of the medical product inserted into the body. Frequently, only a temporary presence of the implant in the body is necessary to fulfil the medical purpose. Implants made of materials which do not degrade in the body are often to be removed again, because rejection reactions of the body may occur in long term even with highly biocompatible permanent materials.

One approach for avoiding additional surgical intervention is to form the implant entirely or in major parts from a biodegradable (or biocorrodible) material. The term biodegradation as used herewith is understood as the sum of microbial procedures or processes solely caused by the presence of bodily media, which result in a gradual degradation of the structure comprising the material. At a specific time, the implant, or at least the part of the implant which comprises the biodegradable material, loses its mechanical integrity. The degradation products are mainly resorbed by the body, although small residues being in general tolerable.

Biodegradable materials have been developed, inter alia, on the basis of polymers of synthetic nature or natural origin. Because of the material properties, but particularly also because of the degradation products of the synthetic polymers, the use of biodegradable polymers is still significantly limited. Thus, for example, orthopedic implants must frequently withstand high mechanical strains and vascular implants, e.g., stents, must meet very special requirements for modulus of elasticity, brittleness, and formability depending on their design.

One promising attempted achievement provides the use of biodegradable metal alloys. For example, it is suggested in German Patent Application No. 197 31 021 A1 to form medical implants from a metallic material whose main component is to be selected from the group of alkali metals, alkaline earth metals, iron, zinc, and aluminium. Alloys based on magnesium, iron, and zinc are described as especially suitable. Secondary components of the alloys may be manganese, cobalt, nickel, chromium, copper, cadmium, lead, tin, thorium, zirconium, silver, gold, palladium, platinum, silicon, calcium, lithium, aluminium, zinc, and iron.

The use of a biodegradable magnesium alloy having a proportion of magnesium greater than 90% by weight, yttrium 3.7 - 5.5% by weight, rare earth metals 1.5 - 4.4% by weight, and the remainder less than 1% by weight is known from European Patent 1 419 793 B1. The material disclosed therein is in particular suitable for producing stents.

Another intravascular implant is described in European Patent Application 1 842 507 A1, wherein the implant is made of a magnesium alloy including gadolinium and the magnesium alloy is being free of yttrium.

Stents made of a biodegradable magnesium alloy arc already in clinical trials. In particular, the yttrium (W) and rare earth elements (E) containing magnesium alloy ELEKTRON WE43 (US 4,401,621) of Magnesium Elektron, UK, has been investigated, wherein a content of yttrium is about 4% by weight and a content of rare earth metals (RE) is about 3% by weight. The following abbreviations are often used: RE = rare earth elements, LRE = light rare earth elements (La-Pm) and HRE = heavy rare earth elements (Sm-Lu): However, it was found that the alloys respond to thermo-mechanical treatments. Although these types of WE alloys originally were designed for high temperature applications where high creep strength was required, it has now been found that dramatic changes in the microstructure occurred during processing with repetitive deformation and heat treatment cycles. These changes in the microstructure are responsible for high scrap rates during production and inhomogencous properties of seamless tubes and therefore in the final product. As a consequence, mechanical properties arc harmfully affected. Especially, the tensile properties of drawn tubes in the process of manufacturing stents are deteriorated and fractures appear during processing. In addition, a large scatter of the mechanical properties especially the elongation at fracture (early fractures of the tubes below yield strength during tensile testing) was found in the final tube. Finally, the in vivo degradation of the stent is too fast and too inhomogeneous and therefore the biocompatibility may be worse due to inflammation process caused by a tissue overload of the degradation products.

The use of mixtures of light rare earth elements (LRE; La, Ce, Pr, Nd) and heavy rare earth metals (HRE; elements of the periodic table: Sm, Eu, Gd, Tb, Dy, Ho, Er, Tm, Yb and Lu) in commercially available magnesium alloys such as WE43 rather than pure alloying elements reduced the costs and it has been demonstrated that the formation of additional precipitates of these elements beside the main precipitates based on Y and Nd further enhance the high temperature strength of the material [King et al, 59th Annual World Magnesium Conference, 2005, p. 15ff]. It could therefore be postulated that the HRE containing precipitates are more stable against growth at higher temperatures because of the significantly slower diffusion rate of these elements compared to Y and Nd. Therefore they contribute substantially to the high temperature strength of WE alloys (particle hardening effect).

However, it now has been found that these HRE precipitates are causing problems when the material is used in biomedical applications, such as vascular implants (e.g. stents) or in orthopaedic implants. The HRE intermetallic particles can adversely affect the thermomcchanical processability of alloys. For example, manufacturing vascular prostheses like stents made of metallic materials usually starts from drawn seamless tubes made of the material. The production of such seamless tubes is usually an alternating process of cold deformation by drawing and subsequent thermal treatments to restore the deformability and ductility, respectively. During the mechanical deformation steps intermetallic particles cause problems because they usually have significant higher hardness than the surrounding matrix. This leads to crack formation in the vicinity of the particles and therefore to defects in the (semi-finished) parts which reduces their usability in terms of further processing by drawing and also as final parts for production of stents.

Surprisingly it now has been found that precipitation still happens to occur although the temperature regime is high enough that one would expect dissolution of all existing particles. This indicates that intermetallic phases predominantly formed with LRE cannot be dissolved during usual recrystallization heat treatment (300 to 525°C) of the specific alloys. As a consequence, the ductility for further deformation processes or service cannot be restored sufficiently.

As mentioned above, magnesium has many advantages for biomedical applications, for example biodegradable inserts like stents, screws/plates for bone repair and surgical suture materials. For many applications however, the time for degradation and failure of the magnesium repair device is too soon and can develop too much gas evolution (H₂) during the corrosion process. Additionally failure of stressed magnesium devices can occur due to Environmentally Assisted Cracking (EAC). EAC also referred to as Stress Corrosion Cracking (SCC) or Corrosion Fatigue (CF) is a phenomenon which can result in catastrophic failure of a material. This failure often occurs below the Yield Strength (YS). The requirement for EAC to occur is three fold: namely mechanical loading, susceptible material, and a suitable environment.

ECSS (European Cooperation for Space Standardisation), quantifies the susceptibility of various metallic alloys by use of an industry recognised test, employing aqueous NaCl solution . ECSS-Q-70-36 report ranks the susceptibility of several Magnesium alloys, including Mg-Y-Nd-HRE-Zr alloy WE54. This reference classifies materials as high, moderate or low resistance to SCC. WE54 is classed as "low resistance to SCC" (ie poor performance). For Biomedical applications, stresses are imposed on the materials and the in vivo environment (e.g. blood) is known to be most corrosive. As for the ECSS tests, SBF (simulated body fluid) widely used for in vitro testing includes NaCl. Tests described in this patent application suggest that EAC performance of the Mg-Y-Nd-HRE-Zr alloy system can be improved by selective use of HRE additions. This offers a significant benefit for biomedical implants, where premature failure could have catastrophic results, for example Atrens (Overview of stress corrosion cracking of magnesium alloys - 8^{th} International conference on Magnesium alloys - DGM 2009), relates potential use of stents in heart surgery, whereby fracture due to SCC would probably be fatal. The consequence of premature failure may include re-intervention, patient trauma, etc. The alloys used must still be formable and show sufficient strength.

An aim of this invention is to overcome or to at least lower one or more of the above mentioned problems. There is a demand for a biodegradable Mg alloy having improved processability especially in new highly sophisticated techniques like micro-extrusion and, if applicable, improved mechanical properties of the material, such as strength, ductility and strain hardening. In particular, in case of the implant is a stent, scaffolding strength of the final device as well as the tube drawing properties of the material should be improved.

A further aspect of the invention may be to enhance the corrosion resistance of the material, more specifically, to slow the degradation, to fasten the formation of a protective conversion layer, and to lessen the hydrogen evolution. In case of a stent, enhancing the corrosion resistance will lengthen the time wherein the implant can provide sufficient scaffolding ability in vivo.

Another aspect of the invention may be to enhance the biocompatibility of the material by avoiding toxic components in the alloy or the corrosion products.

One or more of the above mentioned aspects can be achieved by the implant of the present invention. The inventive implant is made in total or in parts of a biodegradable magnesium alloy comprising:
Y: 0 - 10.0% by weight
Nd: 0 - 4.5% by weight
Gd: 0 - 9.0% by weight
Dy: 0 - 8.0% by weight
Ho: 0 - 19.0% by weight
Er: 0 - 23.0% by weight
Lu: 0 - 25.0% by weight
Tm: 0 - 21.0% by weight
Tb: 0 - 21.0% by weight
Zr: 0.1 - 1.5% by weight
Ca: 0 - 2.0% by weight
Zn: 0 - 1.5% by weight
In: 0 - 12.0% by weight
Sc: 0 - 15.0% by weight
incidental impurities up to a total of 0.3% by weight
the balance being magnesium and under the condition that
a) a total content of Ho, Er, Lu, Tb and Tm is more than 5.5% by weight;
b) a total content of Y, Nd and Gd is more than 2% by weight; and
c) a total content of all alloy compounds except magnesium is more than 8.5% by weight.

In alternative, the inventive implant is made in total or in parts of a biodegradable magnesium alloy consisting of:
Y: 0-10.0% by weight
Nd: 0 - 4.5% by weight
Gd: 0 - 9.0% by weight
Dy: 0 - 8.0% by weight
Ho: 0 - 19.0% by weight
Er: 0 - 23.0% by weight
Lu: 0 - 25.0% by weight
Tm: 0 - 21.0% by weight
Tb: 0 - 21.0% by weight
Zr: 0.1 - 1.5% by weight
Ca: 0 - 2.0% by weight
Zn: 0 - 1.5% by weight
In: 0 - 12.0% by weight
Sc: 0 - 15.0% by weight
incidental impurities up to a total of 0.3% by weight
the balance being magnesium and under the condition that
   a) a total content of Ho, Er, Lu, Tb and Tm is more than 5.5% by weight;
   b) a total content of Y, Nd and Gd is more than 2% by weight; and
   c) a total content of all alloy compounds except magnesium is more than 8.5% by weight.

The use of the inventive Mg alloy for manufacturing an implant causes an improvement in processability, an increase in corrosion resistance and biocompatibility compared to conventional magnesium alloys, especially WE alloys such as WE43 or WE54.

**Tab. 1: Solid solubility of various LRE and HRE in magnesium**

| Atomic number | Element | RT | 300°C | 400°C | 500°C |
|---|---|---|---|---|---|
| 71 | Lu | 10-12 | 19.5 | 25 | 35 |
| 70 | Yb | ca. 0 | 0.5 | 1.5 | 3.3 |
| 69 | Tm | 10-12 | 17.6 | 21.7 | 27.5 |
| 68 | Er | 10-12 | 18.5 | 23 | 28.3 |
| 67 | Ho | 8-10 | 15.4 | 19.4 | 24.2 |
| 66 | Dy | ca. 5 | 14 | 17.8 | 22.5 |
| 65 | Tb | 1-2 | 12.2 | 16.7 | 21.0 |
| 64 | Gd | ca. 0 | 3.8 | 11.5 | 19.2 |
| 63 | Eu | 0 | 0 | 0 | 0 |
| 62 | Sm | ca. 0 | 0.8 | 1.8 | 4.3 |
| 61 | Pm | -- | -- | -- | -- |
| 60 | Nd | ca. 0 | 0.16 | 0.7 | 2.2 |
| 59 | Pr | ca. 0 | 0.05 | 0.2 | 0.6 |
| 58 | Cc | ca. 0 | 0.06 | 0.08 | 0.26 |
| 57 | La | ca. 0 | 0.01 | 0.01 | 0.03 |
| 39 | Y | 1-2 | 4.2 | 6.5 | 10.0 |
| 21 | Sc | ca. 12 | 12.8 | 15.7 | 18.8 |

The solubility of RE in magnesium varies considerably; see Table 1. It may be expected from one skilled in the art, that the volume of coarse particles present would be primarily related to the Nd content, due to the low solid solubility of this element. Therefore the amount of RE addition may be expected to affect the amount of retained clusters and particles present in the microstructure.

Examination of the microstructure of some of the inventive magnesium alloys and conventional WE43 revealed that for specific compositions there were significant less and smaller precipitates in the inventive magnesium alloy than in WE43.

In other words, the selection of the type of RE, present in Mg alloy, has surprisingly led to an improvement in the formability characteristics although the total amount of RE is significantly increased. It is proposed, that this improvement is achieved by a reduction in hard particles (precipitates).

The content of Y in the Mg alloy is 0 - 10.0% by weight. Preferably, the content of Y in the Mg alloy is 1.0 - 6.0% by weight; most preferred 3.0 - 4.0% by weight. Keeping the content of Y within the ranges ensures that the consistency of properties, e.g. scatter during tensile testing, is maintained. Further, strength and corrosion behaviour is improved. When the content of Y is above 10.0% by weight, the ductility of the alloy is deteriorated.

The content of Nd in the Mg alloy is 0 - 4.5% by weight, preferably 0.05 - 2.5% by weight. When the content of Nd is above 4.5% by weight, the ductility of the alloy is deteriorated due to a limited solubility ofNd in Mg.

The content of Gd in the Mg alloy is 0 - 9.0% by weight, preferably 0 - 4.0% by weight. Gd can reduce the degradation of the alloy in SBF tests and improve its EAC behaviour. Levels of Gd approaching the solubility limit in a given alloy reduce ductility.

A total content of Y, Nd and Gd in the Mg alloy is more than 2.0% by weight, preferably more than 3.0% by weight.
The content of Dy in the Mg alloy is 0 - 8.0% by weight, preferably 0 - 6.0% by weight, most preferred 0 - 4.0% by weight.

The content of Ho in the Mg alloy is 0 - 19.0% by weight, preferably 4.0 - 15.0% by weight, most preferred 6.0 - 14.0% by weight. Ho can reduce the degradation of the alloy in SBF and increases strength.

The content of Er in the Mg alloy is 0 - 23.0% by weight, preferably 4.0 - 15.0% by weight, most preferred 6.0 - 14.0% by weight. Er can reduce the degradation of the alloy in SBF tests and improve its EAC behaviour and strength.

The content of Lu in the Mg alloy is 0 - 25.0%) by weight, preferably 4.0 - 15.0% by weight, most preferred 6.0 - 14.0% by weight. Lu can reduce the degradation of the alloy in SBF tests and improve its EAC behaviour and strength.

The content of Tm and/or Tb in the Mg alloy is 0 - 21.0% by weight, preferably 4.0 - 15.0% by weight, most preferred 6.0 - 12.0% by weight. For Tb and Tm the same effect on degradation of the alloy and improvement of the EAC behaviour and strength is expected.

A total content of Ho, Er, Lu, Tb and Tm in the Mg alloy is more than 5.5% by weight. Preferably, the total content of Ho, Er, Lu, Tb and Tm in the Mg alloy is 6.5 - 25.0% by weight, most preferred 7.0 - 15.0% by weight. Preferably the total content includes Dy as additional element.

In addition, the content of Zr in the Mg alloy is 0.1 - 1.5% by weight, preferably 0.2 - 0.6% by weight, most preferred 0.2 - 0.4% by weight. For magnesium-zirconium alloys, zirconium has a significant benefit of reducing the grain size of magnesium alloys, especially of the pre-extruded material, which improves the ductility of the alloy. Further, Zr removes contaminants from the melt.

The content of Ca in the Mg alloy is 0 - 2.0% by weight, preferably 0 - 1.0% by weight, most preferred 0.1 - 0.8% by weight. Ca has a significant benefit of reducing the grain size of magnesium alloys.

The content of Zn in the Mg alloy is 0 - 1.5% by weight, preferably 0 - 0.5% by weight, most preferred 0.1 - 0.3% by weight. Zn can contribute to precipitation and can also affect general corrosion.

The content of In in the Mg alloy is 0 - 12.0% by weight, preferably 0 - 2.5% by weight, most preferred 0.0 - 0.8% by weight. In has a benefit of improving the corrosion performance of magnesium alloys. Additionally In has a benefit of reducing the grain size of magnesium alloy.

A total content of In, Zr, Ca and Zn in the Mg alloy is preferably in the range of 0.2 - 2.0% by weight, preferably 0.2 - 0.8% by weight.

The content of Sc in the Mg alloy is 0 - 15% by weight. Sc can have a positive effect on corrosion resistance.

The total content of impurities in the alloy should be less than 0.3% by weight, more preferred less that 0.2% by weight. In particular, the following maximum impurity levels should be preserved:
- Fe, Si, Cu, Mn, and Ag each less than 0.05% by weight
- Ni less than 0.006% by weight
- La, Ce, Pr, Sm, Eu and Yb less than 0.15% by weight, preferably less than 0.1% by weight

For purposes of the present invention, alloys are referred to as biodegradable in which degradation occurs in a physiological environment, which finally results in the entire implant or the part of the implant formed by the material losing its mechanical integrity. Artificial plasma, has been previously described according to EN ISO 10993-15:2000 for biodegradation assays (composition NaCl 6.8 g/l. CaCl₂ 0.2 g/l. KCl 0.4 g/l. MgSO₄ 0.1 g/l. Na-HCO₃ 2.2 g/l. Na₂HPO₄ 0.126 g/l. NaH₂PO₄ 0.026 g/l), is used as a testing medium for testing the corrosion behaviour of an alloy coming into consideration. For this purpose, a sample of the alloy to be assayed is stored in a closed sample container with a defined quantity of the testing medium at 37°C. At time intervals-tailored to the corrosion behaviour to be expected-of a few hours up to multiple months, the sample is removed and examined for corrosion traces in a known way.

Implants are devices introduced into the human body via a surgical method and comprise fasteners for bones, such as screws, plates, or nails, surgical suture material, intestinal clamps, vascular clips, prostheses in the area of the hard and soft tissue, and anchoring elements for electrodes, in particular, of pacemakers or defibrillators. The implant is preferably a stent. Stents of typical construction have filigree support structures made of metallic struts which are initially provided in an unexpanded state for introduction into the body and are then widened into an expanded state at the location of application.

Vascular implants, especially stents, are preferably to be designed in regard to the alloys used in such a way that a mechanical integrity of the implant is maintained for 2 through 20 weeks. Implants as an occluder are preferably to be designed in regard to the biodegradable in such a way that the mechanical integrity of the implant is maintained for 6 through 12 months. Orthopedic implants for osteosynthesis are preferably to be designed in regard to the magnesium alloy in such a way that the mechanical integrity of the implant is maintained for 6 through 36 months.

The present disclosure is explained in greater detail in the following on the basis of exemplary embodiments and the associated drawings.
- FIG. 1 - 7: show microstructures of samples; and
- FIG. 8: shows an example of secondary cracking caused by EAC in SBF solution.
- FIG. 9: shows the evolution of the relative collapse pressure of stents during corrosion fatigue testing.
- FIG. 10: shows the evolution of the relative load bearing cross section of stents during corrosion fatigue testing.

Several melts with different alloy compositions were melted cast, and extruded and subsequently subject to different investigation with the emphasis on the microstructure (grain size, size, fraction and composition of precipitates), the respective thermo-mechanical properties (tensile properties) and the corrosion behaviour with and without superimposed mechanical load. In addition biocompatibility tests were carried out. In general, melts were carried out according to the following casting technique:

High-purity starting materials (≥99.9%) were melted in steel crucibles under a protective gas (CO₂/2% SF₆). The temperature was raised to 760°C to 800°C before the melt was homogenized by stirring. The melt was cast to form bars with a nominal diameter of 120 mm and a length of 300 mm. Next the bars were machined to a nominal diameter of 75 mm with a length of 150 mm to 250 mm and homogenized for 4-8 hours at approximately 525°C.

The material was then heated to 350-500C and extruded with the help of a hydraulic press. The resulting round rods had a diameter in the range of 6 mm to 16 mm, mostly 9.5-12.7 mm. For the following investigations, pieces from the start and end of an extrusion 30 cm long were usually removed.

Table 2 summarises the chemical compositions, corrosion rates and tensile properties of exemplary Mg alloys. MI0007, MI0034 and DF4619 are comparative examples of WE43 within AMS4427 chemical specification used as reference material. Each time, melts were produced to generate tensile data and for metallography.

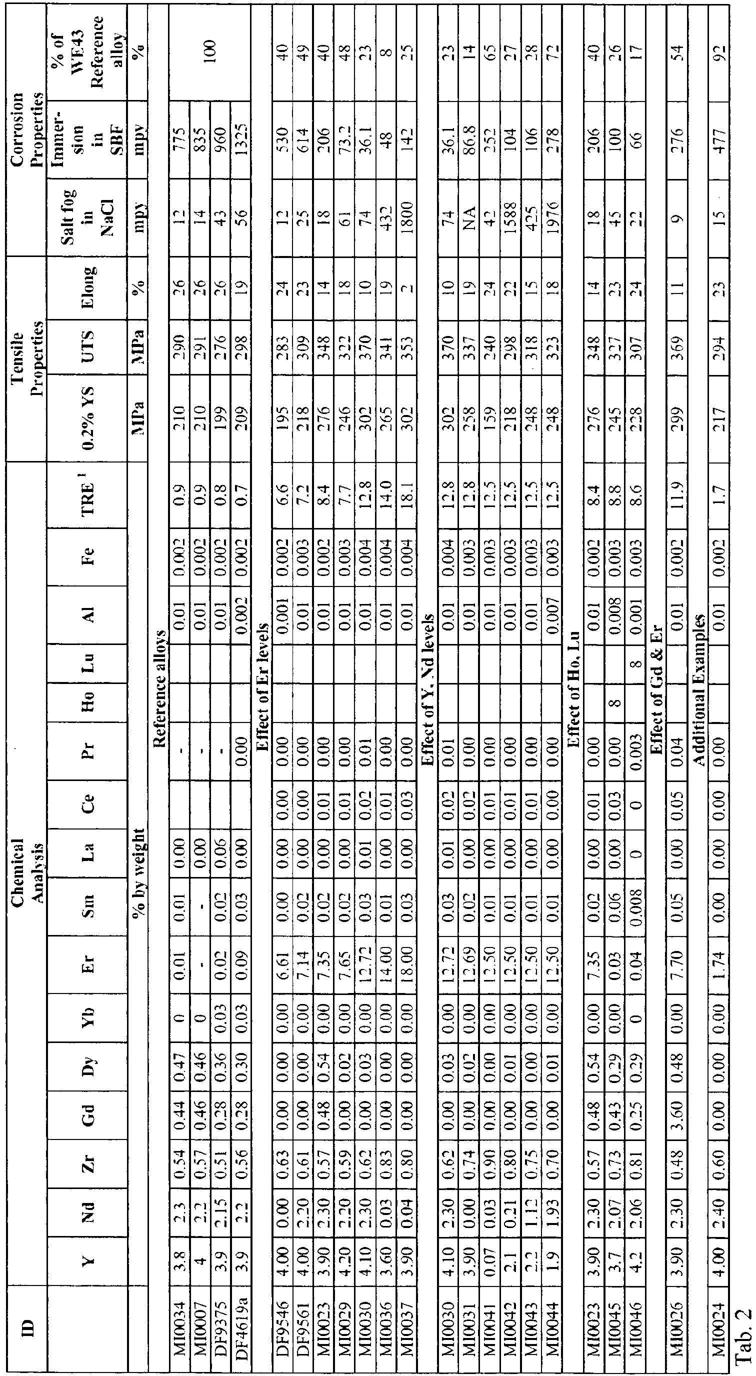

### Mechanical properties and metallurgical description

To determine the mechanical properties, standardized tension tests of the bulk materials were performed and analyzed using several samples of a melt in each case. The 0.2% yield tensile strength (YTS), the ultimate tensile strength (UTS) and elongation at fracture (A) were determined as characteristic data. The yield strength YS of a material is defined as the stress at which material strain changes from elastic deformation to plastic deformation, causing it to deform permanently. The ultimate tensile strength UTS is defined as the maximum stress a material can withstand before break.

In addition tensile test were also performed with extruded tubes and drawn tubes as reference. The typical extruded tubes have a typical length of not less than 30 mm, a diameter of ca. 2 mm and a wall thickness between 50 and 400 µm. They are processed by a hot micro extrusion process at temperatures between 200°C and 480°C and extrusion speeds of 0.1 mm/s to 21 mm/s.

For the metallographic examination of the as extruded condition the materials were melted, cast, homogenized, cut to billets and extruded to bars. Then samples were cut, embedded in epoxy resin, ground, polished to a mirror like finish and etched according to standard metallographic techniques [G Petzow, Metallographisches, keramographisches und plastographisches Ätzen, Borntraeger 2006].

### Discussion of bulk material mechanical properties

Table 2 summarizes the chemical composition, mechanical (tensile test) and corrosion (salt fog in NaCl and immersion in SBF) properties of Mg alloys. As can be seen form the data of Table 2, the inventive changes in the composition of the alloys affect the tensile properties compared to the reference in terms of strength and ductility.

One skilled in the art may expect increasing strength and decreasing ductility with increasing amount of appropriate alloying elements. This can actually be observed in Table 2.

For example, increasing Er content in the approximate range >2% to 8% increases strength and maintains ductility. For higher values of Er, ductility is seen to decline. Reduction in other elements, for example Nd can compensate for the Er addition, minimising the effect of Er on ductility and allowing higher additions of Er to be added; for example MI0036 shows an example of good ductility with 14 % Er addition.

Small changes in other RE additions may affect ductility - for example Gd and Dy (MI0023 vs. DF9561).

Similar effects may be seen for major additions of other REs, for example Ho, Lu, and Gd however different threshold values (compared with Er) are suggested before ductility falls.

It is expected from the data, that higher levels (than the 8% examples shown) of, for example Ho and Lu, could be employed without loss of significant ductility, probably to higher values than the equivalent Er containing examples.

As can be seen from the data of Table 2 the inventive changes of the amount of Y and Nd in the composition of the alloys basically effects strength, ductility and tolerance of some other REs.

Combinations of REs (for example Gd and Er) are not always synergistic; however, certain combinations arc expected to be beneficial.

### Comparing mechanical properties of bulk material and micro-extruded tubes

**Tab. 3: Mechanical properties of extruded bulk material and respective micro-extruded tubes**

| | extruded bulk | | | | extruded bulk +micro-extruded | | |
|---|---|---|---|---|---|---|---|
| ID | YTS in MPa | UTS In MPa | A in % | | YTS in MPa | UTS in MPa | A in % |
| DF9375 Reference | 199 | 276 | 26 | | 164 | 250 | 20 |
| DF9546 | 195 | 283 | 24 | | 173 | 261 | 29 |
| DF9561 | 218 | 309 | 23 | | 189 | 316 | 26 |
| MI0023 | 276 | 348 | 14 | | 227 | 364 | 20 |
| MI0012 | 299 | 369 | 11 | | 206 | 361 | 16 |

For applications, the extruded bulk material is often processed further to achieve a product. This processing can include drawing, rolling and bending steps and other advanced processing techniques. It has now been discovered that surprisingly, alloys of the invention show an improvement during such subsequent processing steps for example micro extrusion.

The comparison of the tensile properties between typical inventive alloys and the reference material before and after micro-extrusion clearly indicate that the inventive alloys are more susceptible to thermo-mechanical treatments, in particular micro-extrusion.

The inventive alloy show a significant drop of 10-30% in yield strength for all tested inventive alloys, minor changes of ca. plus minus 10% in ultimate strength depending on the inventive alloy and significant rise of 10 -50% in ductility for all tested inventive alloys.

All of these effects are desirable because the effect of a lower YS combined with more or less the same UTS leads to a significantly lower (minus 5-30%) yield-to-tensile strength ratio of less than 0.6 which together with the higher ductility is beneficial, for example, in terms of developing stent designs with for example homogenous opening behaviour and higher radial strength.

The reference material in contrast exhibits about 20% drop in yield strength, about 10% drop in ultimate strength and about 20% drop in ductility.

### Microstructure

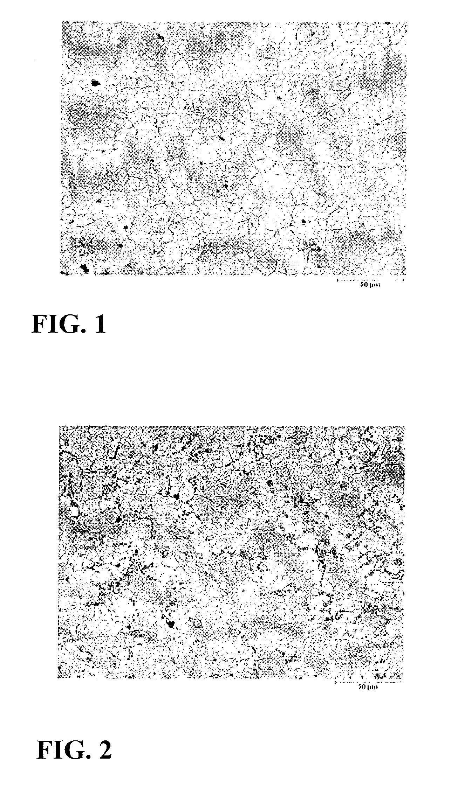

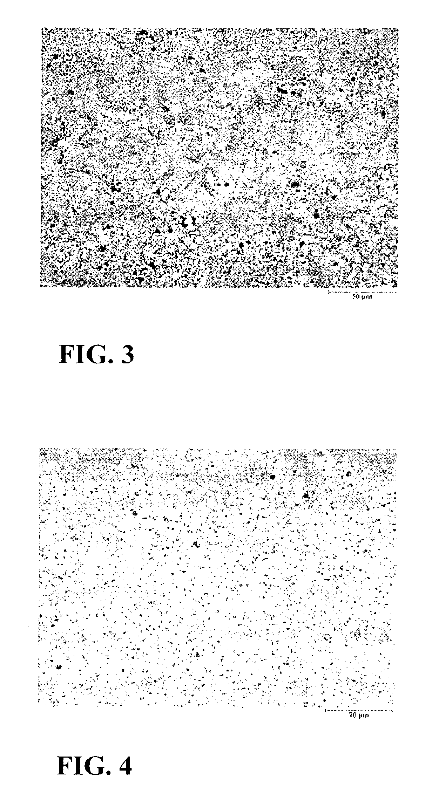

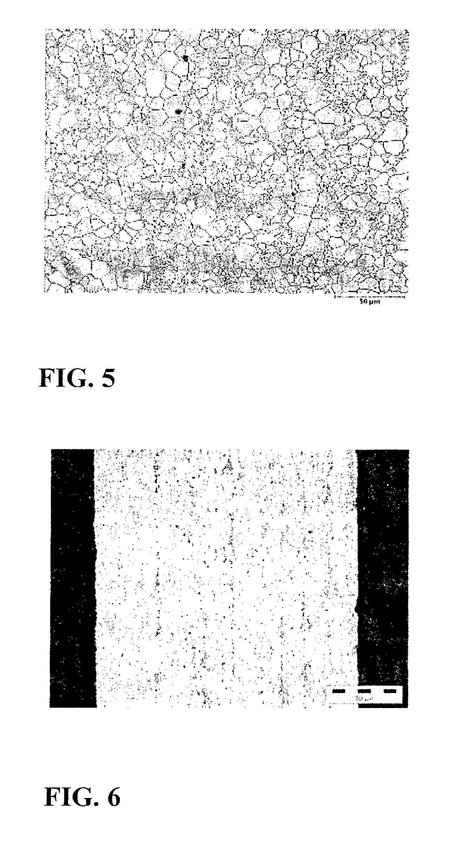

Figures 1 through 5 show microstructures of exemplary samples (FIG. 1: MI0031 / FIG. 2: MI0030 / FIG. 3: MI0037 / FIG. 4: MI0029 / FIG. 5: MI0046) after extrusion. They provide an insight into the effect of alloy composition upon strength and ductility of some of the alloys examples. A microstructure which is free of large particles and clusters ("clean microstructure") can offer the advantage of improved ductility if the clusters/particles are brittle.

Figure 1 is a comparatively "clean microstructure" despite a 12.7% addition of Er - ductility is good (19%).

Figure 2 shows the effect of adding Nd to the alloy of Figure 1. The microstructure has more clusters and ductility falls (10%). It will however be noticed that the alloy of Figure 1 possess higher tensile properties.

Figure 3 contains a higher level of Er (18%) than the alloy of Figure 1. This result in more clusters and despite an improvement in strength, ductility falls to a very low level (2%).

The alloy of Figure 4 illustrates that a combination of lower Er compared to the alloy of Figure 1 (8% Er vs. 13% Er) can achieve a comparatively "clean microstructure" and similar properties to that of alloy of Figure 1, by combining Nd with this lower Er content.

Figure 5 illustrates the effect of Lu, which appears to provide a similar manner to Er, however appears more tolerant to Nd additions in terms of freedom of particles and clusters compared with the alloy of Figure 4.

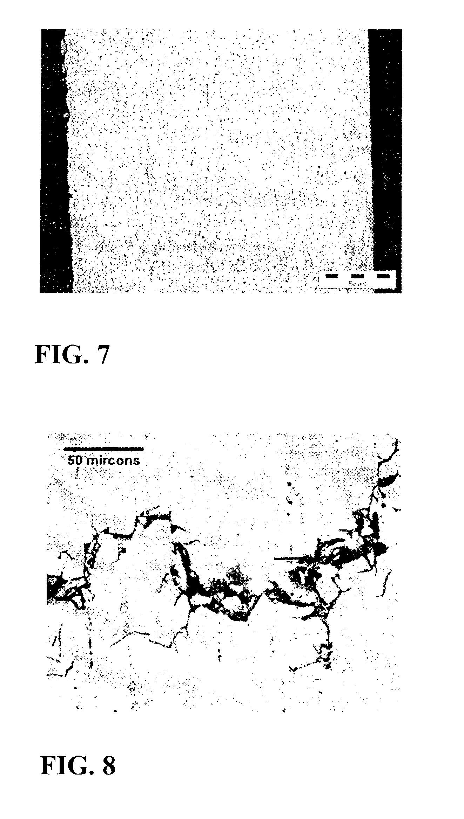

Figure 6 and 7 illustrate the difference in micro-structure of drawn tubes from the reference material and micro-extruded tubes from the inventive alloy MI0029. It clearly can be seen that the micro-extruded has significantly less and smaller precipitates than drawn material. In addition the grain size of the extruded tubes is significantly reduced from ca. 15-20 µm for the as extruded bulk materials and 2-15 µm for the drawn condition.

In structural terms it has been found that an improvement in processability and/or ductility becomes noticeable when the area percentage of particles in the alloy having an average particle size in the range of 1 to 15 µm is less than 3%, and particularly less than 1.5%. Most preferred the area percentage of particles having an average size greater than 1 µm and less than 10 µm is less than 1.5%. These detectable particles tend to be brittle.

In structural terms it has been found that an improvement in processability and/or ductility and/or strength becomes noticeable when the grain size is reduced.

### Corrosion behavior

The corrosion behavior of selected alloy systems was investigated in greater detail on the basis of three standardized tests. The results of these tests are summarized in Table 2 and Table 4.

### Salt Fog Test

First a standardized test to evaluate the industrial usability of the alloys was performed using a 5% NaCl-containing spray mist according to ASTM B117. The samples were exposed to the test conditions for the required number of days and then the corrosion product was removed by boiling in a 10% chromium trioxide solution. The weight loss of the samples was determined and expressed in mpy (mils penetration per year) as is customary in international practice.

### Immersion in SBF

The corrosion resistance also depends on the corrosion medium. Therefore, an additional test method has been used to determine the corrosion behavior under physiological conditions in view of the special use of the alloys.

For storage in SBF (simulated body fluid) with an ionic concentration of 142 mmol/L Na⁺, 5 mmol/L K⁺, 2.5 mmol/L Ca²⁺, 1 mmol/l Mg²⁺, 1 mmol/l SO₄²⁻, 1 mmol/l HPO₄²⁻, 109 mmol/l Cl⁻ and 27 mmol/L HCO₃⁻ cylindrical samples of the extruded material are completely immersed in the hot medium for 7 days at nominally 37°C. The corrosion product is then removed by boiling in a 10% chromium trioxide solution. As for the ASTM B117 test, the weight loss of the samples was determined and expressed in mpy.

An important factor to note is that the absolute value can vary with each batch test. This can make comparison of absolute values difficult. To resolve this, a standard (known reference type alloy WE43 (for example MI0034 type alloy) is tested with each batch of alloys tested. The reference is then used as a basis to compare any improvements. Reference is given the value 100% and values less than this show an improvement (less degradation).

However, the corrosion resistance also depends on corrosion medium and mechanical load conditions acting at the same time. Therefore, an additional test method has been used to determine the Stress Corrosion Cracking (SCC) behavior under physiological conditions in view of the special use of the alloys.

### EAC/SCC in SBF

Stress tests in SBF media were carried out to identify susceptibility to Environmental Assisted Cracking (EAC) also known as stress corrosion cracking (SCC) and compare the alloys of the invention to WE43 type reference alloy.

The test consists of testing a machined cylindrical specimen containing sharp notches to act as stress initiators. The samples were loaded with a fixed weight via a cantilever mechanism. The specimen was located inside a container which allowed SBF media to immerse the sample to a level greater than the notched portion of the sample. Media was changed every two days to minimize any compositional changes during testing. Pass criteria was at least 250 hours continuous exposure to SBF media without failure. The stress value whereby failure occurred in ≥ 250 hours was defined as the threshold value which is reported in Table 4.

To determine the susceptibility of the alloy tested to the SBF media. Each batch was tested to failure in air. This value was compared with the threshold stress value in SBF as described above and the reduction in failure stress expressed as a % of "notched strength in air". It is likely that closer the value is to 100%, the less susceptible the material is to EAC.

### Mg-Ion release from micro-extruded tubes and fully processed stents

However, since it is also known that the thermo-mechanical treatments and the surface conditions of materials affects the corrosion behaviour we also characterized the corrosion resistance of the materials by quantification of the Mg ion release from micro-extruded tubes and actual fully processed stents in SBF.

The samples for the Mg ion release tests were manufactured from micro-extruded tubes as described above. Furthermore the extruded tubes were laser beam cut to the shape of stents, electro-polished, crimped on balloon catheters, sterilized and expanded into hoses of appropriate size where they were surrounded be flowing SBF. Samples from the test solution were taken at different time points and subject to quantitative Mg ion evaluation by means of an ion chromatographic procedure described elsewhere. Drawn tubes of WE43 and the respective stent served as reference.

### Results of Salt fog test

Surprisingly the results of the salt fog test in NaCl atmosphere clearly indicate that an increasing content of Er (Ho, Lu, Tb, Tm) reduces the corrosion resistance significantly. The more surprising is the oppositional corrosion behaviour of the inventive alloys in SBF, a solution simulating the actual biological service environment of vascular implants much better than the salt fog test, since previous investigations indicated distinct correlation between mass loss in salt fog (ASTM B117) and mass loss in SBF. The SBF immersion test revealed a significant reduction of the mass loss with increasing content of Er from 6 wt% to 14 wt%. From about 18 wt% Er on the corrosion rate deteriorates further.

### Results of Immersion in SBF

Tests in immersed SBF, of the alloys of the invention, illustrates that the reduction on degradation rate (corrosion). This is best viewed as a % of the reference alloy. In the best case examples from the invention show a greater than 10 fold improvement in degradation.

Generally speaking, as Er, Ho, Lu, Gd (as well as for Tb and Tm) increase, the degradation resistance also improves, i.e. the measured mass loss becomes lower compared to the reference. Within the alloy additions mentioned above, there also appears to be some differences between the elements individually, with some showing better performance. It would be expected that combinations of some of the HREs could provide synergistic benefits at appropriate alloy contents.

**Tab. 4**

| **ID** | **Chemical Analysis** | | | | | | | | | | | | | | | | **Corrosion Properties** | **EAC Properties** | |
|---|---|---|---|---|---|---|---|---|---|---|---|---|---|---|---|---|---|---|---|
| | **Y** | **Nd** | **Zr** | **Gd** | **Dy** | **Yb** | **Er** | **Sm** | **La** | **Ce** | **Pr** | **Ho** | **Lu** | **Al** | **Fe** | **TRE ¹** | **in SBF** | **UTSin SBF** | **UTS in SBF compared with UTS in air** |
| | **% by weight** | | | | | | | | | | | | | | | | **% of WE43 Reference alloy** | **MPa** | **%** |
| **Reference alloy** | | | | | | | | | | | | | | | | | | | |
| MI0047 | 4.00 | 2.2 | 0.58 | 0.44 | 0.5 | 0.00 | 0.01 | 0.00 | 0.00 | 0.00 | 0.00 | | | 0.01 | 0.002 | 1.0 | 100 | 243 | 60 |

| **Experimental Alloys** | | | | | | | | | | | | | | | | | | | |
|---|---|---|---|---|---|---|---|---|---|---|---|---|---|---|---|---|---|---|---|
| MI0046 | 4.2 | 2.1 | 0.82 | 0.25 | 0.29 | 0.00 | 0.01 | 0.01 | 0.00 | 0.00 | 0.00 | | 8 | 0.01 | 0.002 | 8.6 | 17 | 302 | 70 |
| MI0031 | 3.90 | 0.00 | 0.74 | 0.00 | 0.02 | 0.00 | 12.69 | 0.02 | 0.00 | 0.02 | 0.00 | | | 0.01 | 0.003 | 12.8 | 14 | 285 | 65 |
| DF9546 | 4.00 | 0.00 | 0.63 | 0.00 | 0.00 | 0.00 | 6.61 | 0.00 | 0.00 | 0.00 | 0.00 | | | 0.001 | 0.002 | 6.6 | 40 | 268 | 65 |
| MI0036 | 3.60 | 0.03 | 0.83 | 0.00 | 0.00 | 0.00 | 14.00 | 0.01 | 0.00 | 0.01 | 0.00 | | | 0.01 | 0.004 | 14.0 | 8 | 285 | 60 |
| DF9561 | 4.00 | 2.20 | 0.61 | 0.00 | 0.00 | 0.00 | 7.14 | 0.02 | 0.00 | 0.00 | 0.00 | | | 0.01 | 0.003 | 7.2 | 49 | 270 | 60 |
| MI0037 | 3.90 | 0.04 | 0.80 | 0.00 | 0.00 | 0.00 | 18.00 | 0.03 | 0.00 | 0.03 | 0.00 | | | 0.01 | 0.004 | 18.1 | 25 | 169 | <45 |
| MI0045 | 3.7 | 2.1 | 0.73 | 0.43 | 0.29 | 0.00 | 0.03 | 0.06 | 0.00 | 0.03 | 0.00 | 8 | | 0.008 | 0.003 | 8.8 | 26 | <182 | <40 |

| **Additional Examples** | | | | | | | | | | | | | | | | | | | |
|---|---|---|---|---|---|---|---|---|---|---|---|---|---|---|---|---|---|---|---|
| DF9400 | 5.50 | 0.00 | 0.35 | 7.19 | 0.00 | 0.00 | 0.02 | 0.05 | 0.00 | - | - | | | | 0.002 | 7.3 | 58 | 210 | 45 |
| MI0033 | AZ91 (8.5% Al,0.6% Zn,0.2%Mn,0.002%Fe) | | | | | | | | | | | | | | | | | <78 | <20 |

### Results of EAC/SCC in SBF

Table 4 provides data on EAC tests. Taking WE43 type alloy (DF9319) as a reference, it can be seen that as the HRE content increase, the absolute tolerable stress increase. This improvement is also seen as a % of the actual strength of the material when tested in air (no SBF media effect). The closer this value is to 100%, the less the fracture is related to the media and therefore the less prone the material is likely to be to EAC (SCC) in that media.

Er additions perform well to at least 14wt %, however at 18 wt % the performance is reduced to beneath that of the reference WE43 type alloy. Other HREs perform in different ways, for example only 4% Gd addition gives the best EAC resistance of the alloys tested, and Lu also appears good, Ho performs poorly.

Figure 8 shows the fracture appearance of alloy DF9400. The fracture shows primary and secondary cracking. This type of cracking with secondary cracking remote from the primary crack can be representative of SCC.

### Discussion: Mg-Ion release from micro-extruded tubes and fully processed stents

**Tab. 5**

| ID | Bulk material | Micro-extruded tube | Stent |
|---|---|---|---|
| | in % of respective reference | | |
| DF9546 | 79 | 52 | 12 |
| MI0029 | 47 | 29 | 49 |
| MI0023 | 42 | 29 | 9 |
| MI0012 | 39 | 62 | 13 |
| MI0026 | 20 | 13 | 12 |

Table 5 shows a comparison of the Mg ion release from bulk material, extruded tubes and the respective stents from these extruded tubes. Values are given in percentage of the respective reference material (reference WE43 bulk materials from Table 2 as reference for the inventive bulk material, drawn tube of WE43 for the extruded tubes of the inventive alloys and stents from drawn tube of WE43 for the stent manufactured from extrude tubes of the inventive alloys).

The Mg ion release tests with micro-extruded tubes and the respective stents as well as with drawn tube and the respective stents as the respective references revealed that the inventive alloys exhibit significantly less Mg ion release (20-80% less Mg ion release than the reference material) indicating a significant higher corrosion resistance. Furthermore the inventive alloys become about 20% to 80% more corrosion resistant than the drawn reference material when processes by micro-extrusion. Further improvements (50-90% less Mg ion release than stents from drawn reference material) can be gained through proper electro-polishing to produce a stent. While the inventive alloys show improved corrosion properties after processing the corrosion properties of the reference material drop during tube-drawing and polishing.

This can be explained by the microstructure of the bulk materials (see section "Mechanical properties and metallurgical description of the alloy") resulting from the inventive changes of the composition and the processed material where the drawn reference tube shows significantly more precipitates than the micro-extruded material. These precipitates (known to be electrochemical more noble) also exist on the polished surface creating galvanic couples that accelerate dissolution rates.

In addition the grain size is significantly reduced from ca. 15-20 µm in the as extruded and drawn condition to 2-15 µm in the micro-extruded condition.

### Example: Mg-4Y-2Nd-8Er-0.6Zr (MI0029) & Mg-4Y-8Er-0.6Zr (DF9546)

High purity (>99.9%) magnesium ingots are smelted in steel crucibles at 500-800°C. The melt is protected from burning and sludge formation using fluxless techniques with mixtures of protective gases, e.g. CO₂/2% SF₆ or argon/2% SF₆. After smelting the pure magnesium ingots the temperature is raised to 680-860°C and the respective amounts of alloy ingredients of Y, Nd and Er and Zr are added.

Before casting in a water-cooled mold to form bars with a nominal diameter of 120 mm and a length of 300 mm the melt is homogenized by stirring. After casting and cooling the bars are machined to a nominal diameter of 75 mm with a length of 250 mm and homogenized for 8 hours at approximately 525°C.

The material is then reheated to 400-500°C, preferably 450°C, and extruded with the help of a hydraulic press. The resulting round rods have a diameter of 12.7 mm. Before further processing or testing, 30 cm long pieces are removed from the start and end of the extrusions. The mechanical properties of the extruded bulk materials are:
MI0029:
   YTS = 246 MPa which is ca. 35 MPa higher than for WE43.
   UTS = 322 MPa which is ca. 30 MPa higher than for WE43.
   E = 18% which is ca. 8% less than for WE43.
   The corresponding microstructure is depicted in Fig. 4.
DF9546:
   YTS = 195 MPa which is ca. 15 MPa less than for WE43.
   UTS = 283 MPa which is ca. 7 MPa less than for WE43.
   E = 24% which is 2% less than for WE43.

In particular for medical applications as vessel scaffolds (also stents) in the vascular field the extruded material must be further processed into tubes, e.g. by drawing or micro-extrusion. A Stent is an endoluminal endoprosthesis having a carrier structure that is formed of a hollow body which is open at its ends and the peripheral wall of which is formed by a plurality of struts connecting together which can be folded in a zig-zag or meander-shaped configuration, where the struts have typical dimensions in width and thickness of 30-450µm

Further processing of the extruded alloys into such above mentioned tubes is accomplished by a micro-extrusion process. For micro-extrusion slugs are machined from the bulk material. These slugs are processed by a hot pressing process at elevated temperatures between 200°C and 480°C and extrusion speeds of 0.001 mm/s to 600 mm/s. Typical dimensions for micro-extruded tubes for vessel scaffolds have length of not less than 30 mm, a diameter of ca. 2 mm and a wall thickness between 50 and 400 µm.

Beside of the already described mechanical and corrosion properties the mechanical properties of the micro-extruded tubes of these particular alloys compared to the WE43 tubes are:
MI0029:
   YTS = 189 MPa which is ca. 25 MPa higher than for drawn WE43 tubes.
   UTS = 316 MPa which is ca. 66 MPa higher than for drawn WE43 tubes.
   E = 26% which is ca. 6% higher than drawn WE43 tubes.
   The corresponding microstructures are given in Fig. 7.
DF9546:
   YTS = 173 MPa which is ca. 10 MPa higher than for drawn WE43 tubes.
   UTS = 261 MPa which is ca. 11 MPa higher than for drawn WE43 tubes.
   E = 29% which is ca. 9% higher than drawn WE43 tubes.

To evaluate the susceptibility to environmental assisted cracking in particular corrosion fatigue upon an in vivo like, cyclic loaded vascular scaffold, we produced actual stents from the micro-extruded tube by laser cutting and electro polishing.

Prior to testing the stents were crimped on balloon catheters to a diameter of less than 1.5 mm and sterilized, e.g. ETO (Ethylene Oxide Sterilization) or e-beam (electron beam sterilization). The stents were than over-expanded to their nominal diameter plus 0.5 mm into mock arteries with respective diameters which were previously filled with simulated body fluid (SBF). Previous tests have shown that over-expansion to about 1 mm in diameter is possible for the new alloy while the same stent manufactured from WE43 tolerates significant less over-expansion. The improved dilatation reserve of the inventive alloys contributes significantly to device safety in clinical practice.

The mock arteries with the stent inside are placed in a test chamber where a cyclic physiological load is applied. After certain periods of time (14 and 28 days) some arteries are transferred into another test chamber where the radial strength of the stent can be measured. Some other arteries are filled with epoxy resin for metallographic determination of the remaining load bearing cross section of the stent struts. For comparison we used the same stent design manufactured from WE43 tubing.

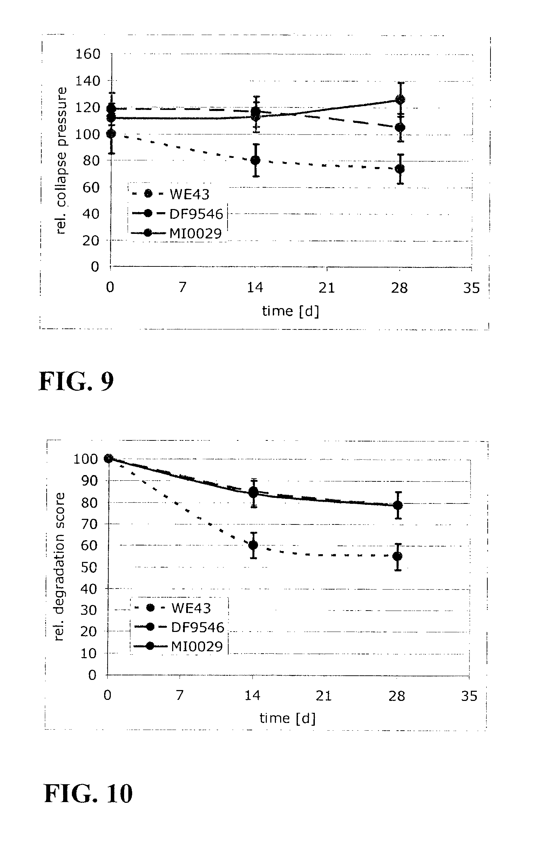

The results of the relative degradation score, which is defined as the percentage of metal remaining during corrosion normalized to the respective value of the reference, depicted in Fig. 10, impressively indicate that the inventive alloys exhibit significantly less uniform corrosion (-25%) than the reference when cyclic loaded in a corrosive environment.

The results of the relative collapse pressure measurement, which is defined as the absolute collapse pressure normalized to the initial collapse pressure of the reference, depicted in Fig. 9, also impressively indicate that stents manufactured from inventive alloys exhibit a significant higher initial collapse pressures (+10%) as an result of the higher strength, the lower yield ratio and the higher strain hardening. Furthermore the stents maintain that high initial level of scaffold ability over a significant longer period of time without fractures or fragmentation, indicating significant less susceptibility to environmental assisted cracking in particular corrosion fatigue.

The positive effect of the addition of highly soluble Er to Mg-Y-Zr and Mg-Y-Nd-Zr-alloys becomes also obvious especially when comparing the microstructure after micro-extrusion with the microstructure of micro-extruded WE43. In Fig. 6 can be seen that a large portion of linear agglomerates of large precipitates (stringers) is present in the WE43 tube (Fig. 7) while the particles are significantly finer and more evenly distributed in the MI0029 tube. Those particles may act as cathodes for galvanic corrosion as well as crack ignition sides during static or cyclic loading and therefore unfavourable.

## Claims

1. Implant made in total or in parts of a biodegradable magnesium alloy comprising:
Y: 0 - 10.0% by weight
Nd: 0 - 4.5% by weight
Gd: 0 - 9.0% by weight
Dy: 0 - 8.0% by weight
Ho: 0 - 19.0% by weight
Er: 0 - 23.0% by weight
Lu: 0 - 25.0% by weight
Tm: 0 - 21.0% by weight
Tb: 0 - 21.0% by weight
Zr: 0.1 - 1.5% by weight
Ca: 0 - 2.0% by weight
Zn: 0 - 1.5% by weight
In: 0 - 12.0% by weight
Sc: 0 - 15.0% by weight
incidental impurities up to a total of 0.3% by weight;
wherein the condition is, that
a) a total content of Ho, Er, Lu, Tb and Tm is equal or more than 5.5% by weight;
b) a total content of Y, Nd and Gd is equal or more than 2% by weight
c) a total content of all alloy compounds except magnesium is equal or more 8.5% by weight; and
d) the balance to 100% by weight being magnesium.

2. The implant of claim 1, wherein the content of Y is 1.0 - 6.0% by weight.

3. The implant of any one of the preceding claims, wherein the content of Nd is 0.05 - 2.5% by weight.

4. The implant of any one of the preceding claims, wherein the content of Gd is 0 - 4.0% by weight.

5. The implant of any one of the preceding claims, wherein a total content of Y, Nd and Gd in the Mg alloy is more than 3.0% by weight.

6. The implant of any one of the preceding claims, wherein the content of Dy is 0 - 6.0% by weight.

7. The implant of any one of the preceding claims, wherein the content of Ho is 4 - 15.0% by weight.

8. The implant of any one of the preceding claims, wherein the content of Er is 4.0 - 15.0% by weight.

9. The implant of any one of the preceding claims, wherein the content of Lu is 4.0 - 15.0% by weight.

10. The implant of any one of the preceding claims, wherein the total content of Dy, Ho, Er, Lu, Tb and Tm is in the range of 6.5 - 25.0% by weight.

11. The implant of any one of the preceding claims, wherein the content of Zr is 0.2 - 0.6% by weight.

12. The implant of any one of the preceding claims, wherein the content of Ca is 0 - 1.0%) by weight.

13. The implant of any one of the preceding claims, wherein the total content of In, Zr, Ca and Zn is in the range of 0.2 - 2.0% by weight.

14. Use of a biodegradable magnesium alloy comprising:
Y: 0 - 10.0% by weight
Nd: 0 - 4.5% by weight
Gd: 0 - 9.0% by weight
Dy: 0 - 8.0% by weight
Ho: 0 - 19.0% by weight
Er: 0 - 23.0% by weight
Lu: 0 - 25.0% by weight
Tm: 0 - 21.0% by weight
Tb: 0 - 21.0% by weight
Zr: 0.1 - 1.5% by weight
Ca: 0 - 2.0% by weight
Zn: 0 - 1.5% by weight
In: 0 - 12.0% by weight
Sc: 0 - 15.0% by weight
incidental impurities up to a total of 0.3% by weight wherein the condition is, that
a) a total content of Ho, Er, Lu, Tb and Tm is equal or more than 5.5% by weight;
b) a total content of Y, Nd and Gd is equal or more than 2% by weight
c) a total content of all alloy compounds except magnesium is equal or more 8.5% by weight; and
d) the balance to 100% by weight being magnesium;
for manufacturing of an implant.

15. Use of claim 14, wherein the implant is a stent.

## Patentansprüche

1. Implantat, das im Ganzen oder in Teilen aus einer biologisch abbaubaren Magnesiumlegierung besteht, die aufweist:
Y: 0 - 10,0 Gew.%
Nd: 0 - 4,5 Gew.%
Gd: 0 - 9,0 Gew.%
Dy: 0 - 8,0 Gew.%
Ho: 0 - 19,0 Gew.%
Er: 0 - 23,0 Gew.%
Lu: 0 - 25,0 Gew.%
Tm: 0 - 21,0 Gew.%
Tb: 0 - 21,0 Gew.%
Zr: 0,1 - 1,5 Gew.%
Ca: 0 - 2,0 Gew.%
Zn: 0 - 1,5 Gew.%
In: 0 - 12,0 Gew.%
Sc: 0 - 15,0 Gew.%
zufällige Verunreinigungen bis zu insgesamt 0,3 Gew.%;
unter der Bedingung, dass
a) ein Gesamtgehalt an Ho, Er, Lu, Tb und Tm gleich oder größer 5,5 Gew.% ist;
b) ein Gesamtgehalt an Y, Nd und Gd gleich oder größer 2 Gew.% ist; und
c) ein Gesamtgehalt aller Legierungskomponenten außer Magnesium gleich oder größer 8,5 Gew.% ist; und
d) der Rest zu 100 Gew.% Magnesium ist.

2. Implantat nach Anspruch 1, wobei der Y-Gehalt 1,0 bis 6,0 Gew.% beträgt.

3. Implantat nach einem der vorangehenden Ansprüche, wobei der Nd-Gehalt 0,05 - 2,5 Gew.% beträgt.

4. Implantat nach einem der vorangehenden Ansprüche, wobei der Gd-Gehalt 0 - 4,0 Gew.% beträgt.

5. Implantat nach einem der vorangehenden Ansprüche, wobei der Gehalt an Y, Nd und Gd in der Mg-Legierung insgesamt über 3,0 Gew.% beträgt.

6. Implantat nach einem der vorangehenden Ansprüche, wobei der Dy-Gehalt 0 - 6,0 Gew.% beträgt.

7. Implantat nach einem der vorangehenden Ansprüche, wobei der Ho-Gehalt 4 - 15,0 Gew.% beträgt.

8. Implantat nach einem der vorangehenden Ansprüche, wobei der Er-Gehalt 4,0 - 15,0 Gew.% beträgt.

9. Implantat nach einem der vorangehenden Ansprüche, wobei der Lu-Gehalt 4,0 - 15,0 Gew.% beträgt.

10. Implantat nach einem der vorangehenden Ansprüche, wobei der Gehalt an Dy, Ho, Er, Lu, Tb und Tm insgesamt im Bereich von 6,5 - 25,0 Gew.% liegt.

11. Implantat nach einem der vorangehenden Ansprüche, wobei der Zr-Gehalt 0,2 - 0,6 Gew.% beträgt.

12. Implantat nach einem der vorangehenden Ansprüche, wobei der Ca-Gehalt 0 - 1,0 Gew.% beträgt.

13. Implantat nach einem der vorangehenden Ansprüche, wobei der Gehalt an In, Zr, Ca und Zn insgesamt im Bereich von 0,2 - 2,0 Gew.% liegt.

14. Verwendung einer biologisch abbaubaren Magnesiumlegierung, die aufweist:
Y: 0 - 10,0 Gew.%
Nd: 0 - 4,5 Gew.%
Gd: 0 - 9,0 Gew.%
Dy: 0 - 8,0 Gew.%
Ho: 0 - 19,0 Gew.%
Er: 0 - 23,0 Gew.%
Lu: 0 - 25,0 Gew.%
Tm: 0 - 21,0 Gew.%
Tb: 0 - 21,0 Gew.%
Zr: 0,1 - 1,5 Gew.%
Ca: 0 - 2,0 Gew.%
Zn: 0 - 1,5 Gew.%
In: 0 - 12,0 Gew.%
Sc: 0 - 15,0 Gew.%
zufällige Verunreinigungen bis zu insgesamt 0,3 Gew.%;
unter der Bedingung, dass
a) ein Gesamtgehalt an Ho, Er, Lu, Tb und Tm gleich oder größer 5,5 Gew.% ist;
b) ein Gesamtgehalt an Y, Nd und Gd gleich oder größer 2 Gew.% ist; und
c) ein Gesamtgehalt aller Legierungskomponenten außer Magnesium gleich oder größer 8,5 Gew.% ist; und
d) der Rest zu 100 Gew.% Magnesium ist,
zur Herstellung eines Implantats.

15. Verwendung nach Anspruch 14, wobei das Implantat ein Stent ist.

## Revendications

1. Implant entièrement ou partiellement constitué d'un alliage de magnésium biodégradable, comprenant :
Y : 0 - 10,0% en poids
Nd : 0 - 4,5% en poids
Gd : 0 - 9,0% en poids
Dy : 0 - 8,0% en poids
Ho: 0 - 19,0% en poids
Er : 0 - 23,0% en poids
Lu: 0 - 25,0% en poids
Tm: 0 - 21,0% en poids
Tb : 0 - 21,0% en poids
Zr : 0,1 - 1,5% en poids
Ca : 0 - 2,0% en poids
Zn : 0 - 1,5% en poids
In : 0 - 12,0% en poids
Sc : 0 - 15,0% en poids
des impuretés accidentelles jusqu'à un total de 0,3% en poids ;
dans lequel la condition est que
a) une teneur totale de Ho, Er, Lu, Tb et Tm est égale ou supérieure à 5,5% en poids ;
b) une teneur totale de Y, Nd et Gd est égale ou supérieure à 2% en poids,
c) une teneur totale de tous les composants de l'alliage sauf le magnésium est égale ou supérieure à 8,5% en poids ; et
d) le reste pour arriver à 100% en poids étant du magnésium.

2. Implant selon la revendication 1, dans lequel la teneur de Y est comprise entre 1,0 et 6,0% en poids.

3. Implant selon l'une quelconque des revendications précédentes, dans lequel la teneur de Nd est comprise entre 0,05 et 2,5% en poids.

4. Implant selon l'une quelconque des revendications précédentes, dans lequel la teneur de Gd est comprise entre 0 et 4,0% en poids.

5. Implant selon l'une quelconque des revendications précédentes, dans lequel une teneur totale de Y, Nd et Gd dans l'alliage Mg est supérieure à 3,0% en poids.

6. Implant selon l'une quelconque des revendications précédentes, dans lequel la teneur de Dy est comprise entre 0 et 6,0% en poids.

7. Implant selon l'une quelconque des revendications précédentes, dans lequel la teneur de Ho est comprise entre 4 et 15,0% en poids.

8. Implant selon l'une quelconque des revendications précédentes, dans lequel la teneur de Er est comprise entre 4,0 et 15,0% en poids.

9. Implant selon l'une quelconque des revendications précédentes, dans lequel la teneur de Lu est comprise entre 4,0 et 15,0% en poids.

10. Implant selon l'une quelconque des revendications précédentes, dans lequel la teneur totale de Dy, Ho, Er, Lu, Tb et Tm est comprise dans la plage de 6,5 à 25,0% en poids.

11. Implant selon l'une quelconque des revendications précédentes, dans lequel la teneur de Zr est comprise entre 0,2 et 0,6% en poids.

12. Implant selon l'une quelconque des revendications précédentes, dans lequel la teneur de Ca est comprise entre 0 et 1,0% en poids.

13. Implant selon l'une quelconque des revendications précédentes, dans lequel la teneur totale de In, Zr, Ca et Zn est comprise dans la plage de 0 à 6,0% en poids.

14. Utilisation d'un alliage de magnésium biodégradable, comprenant :
Y : 0 - 10,0% en poids
Nd : 0 - 4,5% en poids
Gd : 0 - 9,0% en poids
Dy : 0 - 8,0% en poids
Ho: 0 - 19,0% en poids
Er : 0 - 23,0% en poids
Lu: 0 - 25,0% en poids
Tm: 0 - 21,0% en poids
Tb : 0 - 21,0% en poids
Zr : 0,1 - 1,5% en poids
Ca : 0 - 2,0% en poids
Zn : 0 - 1,5% en poids
In : 0 - 12,0% en poids
Sc : 0 - 15,0% en poids
des impuretés accidentelles jusqu'à un total de 0,3% en poids,
dans laquelle la condition est que
a) une teneur totale de Ho, Er, Lu, Tb et Tm est égale ou supérieure à 5,5% en poids;
b) une teneur totale de Y, Nd et Gd est égale ou supérieure à 2% en poids,
c) une teneur totale de tous les composants de l'alliage sauf le magnésium est égale ou supérieure à 8,5% en poids ; et
d) le reste pour arriver à 100% en poids étant du magnésium pour la fabrication d'un implant

15. Utilisation selon la revendication 14 dans laquelle l'implant est un stent.
